(19) Europäisches Patentamt
European Patent Office
Office européen des brevets

(11) EP 1 037 891 B1

(12) **FASCICULE DE BREVET EUROPEEN**

(45) Date de publication et mention de la délivrance du brevet:
**10.04.2002 Bulletin 2002/15**

(21) Numéro de dépôt: **98958961.9**

(22) Date de dépôt: **07.12.1998**

(51) Int Cl.⁷: **C07D 453/02**

(86) Numéro de dépôt international:
**PCT/FR98/02638**

(87) Numéro de publication internationale:
**WO 99/29692 (17.06.1999 Gazette 1999/24)**

(54) **PROCEDE DE PREPARATION DE LA MEQUITAZINE ET INTERMEDIAIRE DE SYNTHESE**

VERFAHREN ZUR HERSTELLUNG VON MEQUITAZIN UND ZWISCHENPRODUKT DER SYNTHESE

METHOD FOR PREPARING MEQUITAZINE AND NOVEL SYNTHESIS INTERMEDIATE

(84) Etats contractants désignés:
**AT BE CH CY DE DK ES FI FR GB GR IE IT LI LU MC NL PT SE**

(30) Priorité: **08.12.1997 FR 9715479**

(43) Date de publication de la demande:
**27.09.2000 Bulletin 2000/39**

(73) Titulaire: **PIERRE FABRE MEDICAMENT**
**92100 Boulogne-Billancourt (FR)**

(72) Inventeurs:
- **GUMINSKI, Yves**
  **F-81090 Lagarrigue (FR)**
- **IMBERT, Thierry**
  **F-81290 Viviers les Montagnes (FR)**
- **LESIMPLE, Patrick**
  **F-81600 Brens (FR)**

(74) Mandataire: **Ahner, Francis et al**
**Cabinet Régimbeau**
**20, rue de Chazelles**
**75847 Paris cedex 17 (FR)**

(56) Documents cités:
**EP-A- 0 089 860** **FR-A- 2 034 605**

- **G.A. EPLING ET AL.: SYNLETT, G.A. EPLING ET AL.: SYNLETT, vol. 5, 1991, pages 347-348, XP002098309 vol. 5, 1991, pages 347-348, XP002098309**
- **CHEMICAL ABSTRACTS, vol. 118, no. 1, CHEMICAL ABSTRACTS, vol. 118, no. 1, 4 janvier 1993 4 janvier 1993 Columbus, Ohio, US; Columbus, Ohio, US; abstract no. 6983, abstract no. 6983, XP002098310 XP002098310 & JP 04 169583 A (YODOGAWA PHARMACEUTICAL & JP 04 169583 A (YODOGAWA PHARMACEUTICAL CO., LTD.) CO., LTD.) cité dans la demande cité dans la demande**
- **CHEMICAL ABSTRACTS, vol. 120, no. 1, CHEMICAL ABSTRACTS, vol. 120, no. 1, 3 janvier 1994 3 janvier 1994 Columbus, Ohio, US; Columbus, Ohio, US; abstract no. 8602, abstract no. 8602, XP002098311 XP002098311 & JP 05 140157 A (SUMIKA FUAIN KEMU KK) & JP 05 140157 A (SUMIKA FUAIN KEMU KK) cité dans la demande cité dans la demande**
- **CHEMICAL ABSTRACTS, vol. 95, no. 17, CHEMICAL ABSTRACTS, vol. 95, no. 17, 26 octobre 1981 26 octobre 1981 Columbus, Ohio, US; Columbus, Ohio, US; abstract no. 150567, abstract no. 150567, XP002076075 XP002076075 & B. CLEMENT ET AL.: ARCH. PHARM., & B. CLEMENT ET AL.: ARCH. PHARM., vol. 314, no. 8, 1981, pages 716-722, vol. 314, no. 8, 1981, pages 716-722,**
- **J.March, ADVANCED ORGANIC CHEMISTRY, 3 ED, J.March, ADVANCED ORGANIC CHEMISTRY, 3 ED, Wiley and Sons, Wiley and Sons,**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 037 891 B1

## Description

La présente invention est relative à un nouveau procédé de préparation de la méquitazine : 10-[1-aza-bicyclo(2,2,2) oct-3-yl] méthyl-10H-phénothiazine de formule 1, ou de son isomère lévogyre de formule 2.

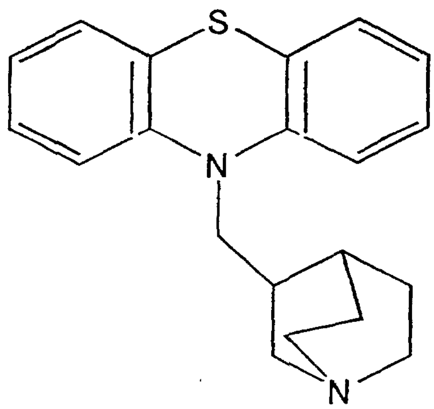

1

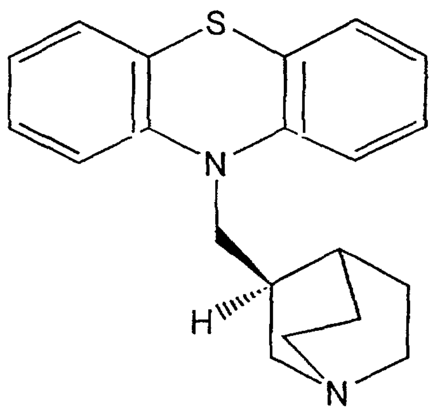

2

La méquitazine 1 est un composé utilisé comme médicament antihistaminique pour le traitement des allergies. Elle est décrite dans le brevet FR-2 034 605 par sa structure chimique et son activité thérapeutique. Son isomère lévogyre 2, seul, possède l'activité biologique désirée. Il est décrit dans le brevet FR-2 522 660 ou EP-089 860. Le procédé de préparation des composés 1 et 2 est également décrit dans ces brevets. Il présente un certain nombre d'inconvénients.

La synthèse se fait, avec un faible rendement, par alkylation du 3-chlorométhyl quinuclidine avec le sel de sodium ou de potassium de la phénothiazine dans un solvant inerte à haut point d'ébullition comme le xylène, éventuellement additionné d'un cosolvant comme le HMPT, toxique. Un autre inconvénient est lié à l'utilisation de l'amidure de sodium qui provoque un dégagement important d'ammoniac gazeux.

La réaction n'est pas propre, un grand nombre de sous-produits est généré. En particulier, le produit d'élimination d'HCl du 3-chlorométhyl quinuclidine en 3-méthylène quinuclidine, est formé dans ces conditions de réaction, ce qui engendre une perte de rendement.

Des produits secondaires sont formés également au moment de la création de l'anion de la phénothiazine, que ce soit avec l'amidure de sodium, l'hydrure de sodium, le tertiobutylate de potassium, etc. D'intenses colorations du milieu réactionnel sont le signe de la délocalisation de l'anion sur les carbones des homocycles benzéniques qui conduit à la formation de sous-produits.

La présence de nombreuses impuretés de la réaction rend nécessaire des traitements chromatographiques, pour isoler la méquitazine pure Enfin, les rendements ainsi obtenus sont faibles.

D'autres brevets ont trait à la préparation de la méquitazine. Par exemple, JP-KOKAI 04 169 583 (Chem. Abstr. : 118 : 6983) et JP-KOKAI 05 140 157 (Chem. Abstr. : 120 : 8602) (Utilisation de l'époxyde spiro de la quinuclidine sur le dérivé potassique de la phénothiazine). Bien que ces brevets soient des améliorations des préparations antérieures, le problème de l'obtention de la méquitazine avec un bon rendement n'est pas résolu.

La présente invention apporte de façon non prévisible la réponse au problème posé de la synthèse de la méquitazine et de son isomère lévogyre.

Cette nouvelle préparation consiste en la formation d'une part du dérivé lithié de la phénothiazine dans un solvant inerte, en particulier le THF, de préférence à environ 0°C, par le butyllithium. Cette opération fournit un milieu réactionnel jaune clair. L'utilisation de ce dérivé lithié présente l'avantage de former l'anion exclusivement sur l'azote de la phénothiazine. Ce procédé est facilement industrialisable, compte tenu des nouvelles technologies de conditionnement du butyllithium. D'autre part, après retour à température ordinaire, on fait réagir sur ce dérivé lithié, le mésylate du 3-hydrométhyl quinuclidine, de formule 3.

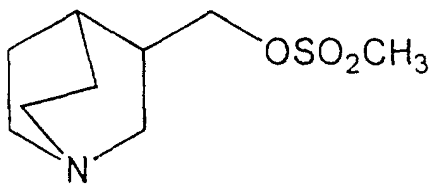

**3**

Ce dérivé n'est pas décrit dans la littérature chimique, et se prépare de façon conventionnelle à partir de son alcool précurseur par action du chlorure de mésyle dans le chloroforme en présence de pyridine, et s'isole sous forme de chlorhydrate.

La réaction du dérivé lithié de la phénothiazine avec le dérivé 3-mésyloxyméthyl quinuclidine est avantageusement conduite dans le THF avec éventuellement de la N-méthylpyrrolidone comme cosolvant polaire aprotique, de préférence à une température d'environ 65°C. Il n'y a pas ou peu de produit d'élimination "3-méthylène exo" quinuclidine. La réaction est relativement rapide (environ 2 heures) et fournit un produit de très grande pureté, puisque après extraction acide-base, le chlorhydrate de la méquitazine cristallise dans l'eau, peut se filtrer et s'obtenir avec un rendement excellent (jusqu'à 90 %).

Les avantages de ce nouveau procédé consistent en :

- l'utilisation du dérivé mésylé de la quinuclidine, ayant une très faible tendance à donner du composé d'élimination : méthylène exo,
- la simplicité de mise en oeuvre,
- la température de réaction moyenne (65°C)
- le milieu réactionnel particulièrement propre pour la formation du dérivé lithié,
- la rapidité de la réaction,
- la pureté du milieu réactionnel après réaction permet de ne pas utiliser de stade chromatographique,
- l'isolement du chlorhydrate de méquitazine est direct,
- le rendement de l'opération est excellent.

La présente invention a également pour objet la préparation de l'isomère lévogyre de la méquitazine. Le dédoublement du 3-hydrométhyl quinuclidine en ses énantiomères est connu par les brevets FR-2 522 660 et EP-089 860. Il est possible de préparer la méquitazine isomère lévogyre par action du (-) 3-hydroxyméthyl quinuclidine pour en faire son dérivé (-) 3-mésyloxyméthyl quinuclidine, puis de faire réagir ce dernier sur le dérivé lithié de la phénothiazine, comme pour le dérivé racémique.

Il est possible également de procéder au dédoublement des énantiomères sur le dérivé 3-mésyloxyméthyl quinuclidine avec l'acide tartrique optiquement actif. Ce dérivé est suffisamment stable pour se prêter à une suite de recristallisations. On obtient alors grâce à l'utilisation de l'acide L-tartrique lévogyre, la cristallisation du (-) 3-mésyloxyméthyl quinuclidine, utilisable pour la réaction avec le dérivé lithié de la phénothiazine de manière analogue au racémique.

L'avantage de séparer les énantiomères au stade du dérivé 3-mésyloxyméthyl quinuclidine est que pendant l'opération de dédoublement, il se produit également une purification chimique. Ceci permet alors d'utiliser un dérivé 3-hydroxyméthyl quinuclidine brut, sans purification, et d'enchaîner l'étape de mésylation puis de dédoublement sans purification intermédiaire, ce qui est industriellement avantageux.

Les modes opératoires suivants sont donnés à titre d'exemple d'illustration de l'invention :

**EXEMPLE 1 : Préparation du 3-mésyloxyméthyl quinuclidine (formule 3)**

A une solution de 50 g de 3-hydroxyméthyl quinuclidine (C.A. Grob et E. Renk, Helv. Chim. Acta 1954, 37, 1689 - 1698) dans 760 ml de chloroforme et 34 ml de pyridine, sont ajoutés 33 ml de chlorure de mésyle goutte à goutte, sous agitation entre 0 et 5°C. L'agitation est maintenue toute la nuit avec retour à température ordinaire. Le chlorhydrate du 3-mésyloxyméthyl quinuclidine précipité dans le milieu réactionnel, est filtré, rincé par l'acétone et l'éther isopropylique, puis séché sous vide. On obtient 73 g, soit 80 % de rendement.

$$F°C = 182°C$$

RMN'H 400 MHz ($CDCl_3$) δ 1,38 (1H,m), 1,48-1,50 (1H,m), 1,57-1,58 (2H,m), 1,79 (1H,s) 2,02-2,05 (1H,m), 2,29-2,34 (1H,dd), 2,70-2,83 (4H,m), 2,95 (3H,s), 2,95-3,02 (1H,m), 4,09-4,17 (2H,m).

**EXEMPLE 2 : Séparation des énantiomères : Préparation du (-) 3-mésyloxyméthyl quinuclidine (isomère lévogyre)**

22 g de (±) 3-mésyloxyméthyl quinuclidine sont placés dans 300 ml d'un mélange éthanol/eau (80/20) avec 15 g d'acide (-) L-tartrique naturel. On porte le mélange réactionnel à reflux jusqu'à dissolution et on laisse revenir à température ordinaire. On observe la cristallisation du tartrate qui est filtré. Les cristaux ainsi obtenus (25,9 g) sont recristallisés 2 fois dans le mélange éthanol/eau (80/20) pour obtenir 14,5 g de tartrate (rendement environ 80 %). La base relarguée par la soude à 30 % fournit :

$$[\alpha]_D^{24°C} = -48,3°(MeOH)$$

**EXEMPLE 3 : Préparation de la méquitazine racémique**

Dans un tricol de 1 litre avec un réfrigérant surmonté d'un ballon d'azote, on introduit 200 ml de THF et 60 g de phénothiazine. Après refroidissement vers 0°C, on introduit 100 ml d'une solution de butyllithium 2,5 M dans l'hexane goutte à goutte. Le milieu réactionnel est agité 1 heure avec retour à température ordinaire. On introduit alors 25,6 g de chlorhydrate de 3-mésyloxyméthyl quinuclidine, obtenu à l'exemple 1 et 100 ml de N-méthyl pyrrolidone. On porte le milieu réactionnel au reflux à 65°C pendant 2 heures. Il est ensuite versé dans 1 litre d'eau et de glace, puis extrait 2 fois par 500 ml d'un mélange acétate d'éthyle/éther isopropylique (50/50). Les phases organiques, réunies, sont lavées 2 fois par 500 ml d'eau, puis par 2 fois 500 ml d'une solution d'acide chlorhydrique 1N.

Les phases aqueuses acides réunies sont à nouveau extraites par un mélange acétate d'éthyle/éther isopropylique (50/50). Le chlorhydrate de méquitazine cristallise alors dans la phase aqueuse acide. Après filtration et lavage du précipité à l'éther isopropylique, on obtient 31 g de cristaux beiges de chlorhydrate de méquitazine (rendement : 87 %). Recristallisation sur un échantillon analytique dans l'alcool isopropylique.

$$F°C = 261°C.$$

**EXEMPLE 4 : Préparation de l'isomère lévogyre de la méquitazine**

Par le même mode opératoire que pour l'exemple 3, mais en partant du dérivé mésylé lévogyre obtenu à l'exemple 2, on obtient l'isomère lévogyre de la méquitazine.

$$F°_{(Base)} = 140°C \qquad [\alpha]_D^{24°C} = -40,5° \text{ (1\% } \textit{éthanol})$$

**Revendications**

1. Procédé de préparation de la méquitazine, **caractérisé en ce que** l'on condense dans un solvant inerte, en présence de butyllithium, le 3-méthanesulfonyloxy méthyl [1-aza-bicyclo(2,2,2)-octane], avec la phénothiazine.

2. Procédé de préparation de l'isomère lévogyre de la méquitazine, **caractérisé en ce que** l'on condense dans un solvant inerte, en présence de butyllithium, l'isomère lévogyre du 3-méthanesulfonyloxyméthyl [1-aza-bicyclo(2,2,2)-octane], avec la phénothiazine.

3. Procédé de préparation selon l'une des revendications 1 et 2, **caractérisée en ce que** la réaction de condensation est conduite à une température de 65°C.

4. Procédé de préparation selon les revendications 1 à 3, **caractérisé en ce que** le solvant inerte est du THF, avec éventuellement de la N-méthyl pyrrolidone comme cosolvant.

5. Composé répondant à la formule 3

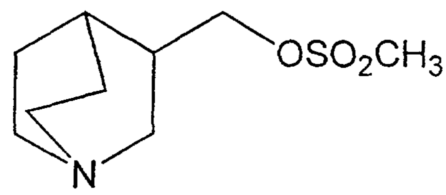

**3**

et ses énantiomères en tant qu'intermédiaires de synthèse pour la préparation de médicaments, en particulier de la méquitazine et de son isomère lévogyre.

**Patentansprüche**

1. Verfahren zur Herstellung von Mequitazin, dadurch'gekennzeichnet, dass man in einem inerten Lösungsmittel in Anwesenheit von Butyllithium 3-Methansulfonyloxymethyl-[1-azabicyclo[2.2.2]octan] mit Phenothiazin kondensiert.

2. Verfahren zur Herstellung des linksdrehenden Isomers von Mequitazin, **dadurch gekennzeichnet, dass** man in einem inerten Lösungsmittel in Anwesenheit von Butyllithium das linksdrehende Isomer von 3-Methansulfonyloxymethyl-[1-azabicyclo [2.2.2]octan] mit Phenothiazin kondensiert.

3. Verfahren zur Herstellung nach einem der Ansprüche 1 und 2, **dadurch gekennzeichnet, dass** die Kondensationsreaktion bei einer Temperatur von 65°C durchgeführt wird.

4. Verfahren zur Herstellung nach den Ansprüchen 1 bis 3, **dadurch gekennzeichnet, dass** das inerte Lösungsmittel THF, gegebenenfalls mit N-Methylpyrrolidon als Cosolvens, ist.

5. Verbindung der Formel 3

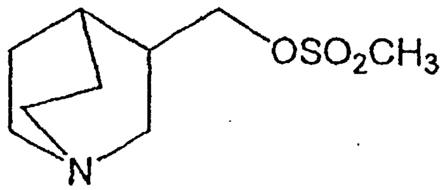

3

und ihre Enantiomere als Synthese-Zwischenprodukte für die Herstellung von Medikamenten, insbesondere von Mequitazin und seinem linksdrehenden Isomer.

## Claims

1. Process for the preparation of mequitazine, **characterized in that** 3-(methanesulphonyloxy-methyl) [1-azabicyclo[2.2.2]octane] is condensed with phenothiazine in an inert solvent in the presence of butyllithium.

2. Process for the preparation of the laevorotatory isomer of mequitazine, **characterized in that** the laevorotatory isomer of 3-(methanesulphonyloxy-methyl)-[1-azabicyclo[2.2.2]octane] is condensed with phenothiazine in an inert solvent in the presence of butyllithium.

3. Preparation process according to either of Claims 1 and 2, **characterized in that** the condensation reaction is carried out at a temperature of 65°C.

4. Preparation process according to Claims 1 to 3, **characterized in that** the inert solvent is THF with optionally N-methylpyrrolidone as cosolvent.

5. Compound corresponding to the formula 3

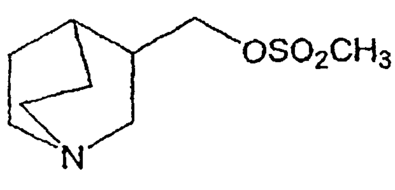

3

and its enantiomers as synthetic intermediates in the preparation of medicaments, in particular of mequitazine and its laevorotatory isomer.